# DEMANDE DE BREVET EUROPEEN

(11) **EP 2 133 346 A1**
(43) Date de publication de la demande: **16.12.2009**
(21) Numéro de dépôt: 08158153.0
(22) Date de dépôt: 12.06.2008
(51) Int. Cl.: C07D 319/12

(54) **Procédé pour la fabrication d'un diester cyclique d'un acide alpha-hydroxylé**

(71) Demandeur: Ktanton Ltd, 34980 Haifa (IL)
(72) Inventeur: Wajc, Samuel J., 34980 Haifa (IL)
(74) Mandataire: Vande Gucht, Anne

(57) **Abrégé**

Procédé pour la fabrication d'un diester cyclique d'un acide α-hydroxylé comprenant les étapes suivantes :
- on mélange un sel substantiellement anhydre d'un métal bivalent et d'un acide α-hydroxylé, avec un acide fort dont le pKa est inférieur à celui de l'acide α-hydroxylé et dont le sel avec le métal bivalent est hygroscopique
- on laisse réagir le mélange pendant une durée suffisante pour obtenir le diester cyclique dispersé dans le sel hygroscopique.

## Description

La présente demande concerne un procédé pour la fabrication d'un diester cyclique d'un acide α-hydroxylé et en particulier, de lactide ou de glycolide. Il concerne en particulier la fabrication de lactide (LD2 ou Lactide Diester) qui est un intermédiaire clé pour la fabrication des polymères d'acide lactique (PLA ou Polylactide), un polymère bio-sourcé (c.à.d. basé sur des ressources naturelles) et biodégradable.

La production du PLA est actuellement en plein développement. Or, les procédés classiques de production du LD2 utilisent l'acide lactique (L1A) comme réactif et se résument à une suite d'étapes difficiles de condensation, de séparation d'eau et de dépolymérisation. Ces étapes sont énergétiquement coûteuses, et le lactide formé n'est pas pur. De plus, le taux de conversion n'est pas supérieur à 60/70% actuellement avec en plus, des sous-produits dont il faut gérer la fin de vie par la suite. La synthèse du lactide est l'étape la plus importante dans le procédé de fabrication classique du PLA. C'est cette étape qui conditionnera le prix du polymère final. Le lactide doit être le plus pur possible pour pouvoir ensuite effectuer la polymérisation par ouverture de cycle.

Le principe à la base de la présente invention consiste à utiliser un lactate (ou un autre sel d'acide α-hydroxylé) que l'on fait réagir avec un acide fort pour libérer de l'acide lactique (ou un autre acide α-hydroxylé) et former un sel déshydratant (hygroscopique). Ce dernier captera in situ les deux molécules d'eau qui seront libérées lors des différentes réactions aboutissant à la formation du diester (et qui seront détaillées plus loin). Le sel déshydratant pourra être valorisé par la suite comme engrais ou pour des applications thérapeutiques. On obtient ainsi un procédé avec des sous-produits utiles et respectueux de l'environnement, qui consomme moins d'énergie que les procédés de l'art antérieur, le recours à un sel déshydratant limitant la température requise pour éliminer l'eau de réaction et évitant en outre l'oligomérisation de l'acide α-hydroxylé.

Pour que ce principe fonctionne, la demanderesse a constaté qu'il fallait que le pKa de l'acide fort soit plus faible que celui de l'acide α-hydroxylé (pour que cet acide fort puisse effectivement réagir avec le sel organique et libérer l'acide α-hydroxylé) et que le sel résultant soit déshydratant.

La présente invention concerne dès lors un procédé pour la fabrication d'un diester cyclique d'un acide α-hydroxylé comprenant les étapes suivantes :
- on mélange un sel d'un métal et d'un acide α-hydroxylé, avec un acide fort dont le pKa est inférieur à celui de l'acide α-hydroxylé et dont le sel avec le métal est hygroscopique
- on laisse réagir le mélange à une température et pendant une durée suffisantes pour obtenir le diester cyclique dispersé dans le sel hygroscopique.

L'acide α-hydroxylé (AHA) dont il est question dans le cadre de l'invention est de préférence obtenu par fermentation de produits naturels tels que les sucres de plantes, fruits... ou les produits laitiers. Il s'agit de préférence d'acide lactique, glycolique, glutarique, mandélique, malique, citrique ou tartrique, les deux premiers étant préférés. L'acide lactique donne de bons résultats.

Le métal dont il est question dans le cadre de l'invention doit être tel que le sel d'acide fort correspondant soit déshydratant; il peut être choisi parmi le Fe, Mg, Zn, Al et Ca. Il s'agit de préférence d'un métal bivalent. Les métaux du type alcalino-terreux sont préférés. Le Ca et le Mg sont particulièrement préférés car pouvant donner, entre autres, des sous-produits valorisables.

Les sels métalliques d'acide α-hydroxylé utilisés dans le procédé selon l'invention ne sont généralement pas disponibles tels quels dans le commerce. Par contre, des hydrates à base de lactate de Mg et de Ca sont disponibles respectivement sous les dénominations commerciales PURAMEX® MG (dihydrate de MgLa2 ou lactate de Mg) et PURACAL® (pentahydrate de CaLa2 ou lactate de Ca). Alternativement, de tels hydrates peuvent être synthétisés par fermentation de sucres ou de produits laitiers à l'aide de bactéries (avec ajout d'une base pour obtenir un pH voisin de (voire légèrement inférieur à) 7), puis purification par cristallisation. Dans le cas des glycolates, on trouve sur le marché de l'acide glycolique que l'on peut faire réagir avec un hydroxyde métallique pour former un hydrate du glycolate correspondant.

Les hydrates susmentionnés sont facilement (au moins partiellement) déshydratés (pour former une poudre de cristaux anhydres) à une température modérée et ce même à pression atmosphérique. Par exemple, un traitement à une température comprise entre 90 et 180°C, de préférence entre 100 et 160°C et de manière tout particulièrement préférée, entre 110 et 130°C donne de bons résultats (généralement même au bout d'une à 2 heures de traitement à pression atmosphérique). Une température trop faible augmente inutilement la durée du traitement et l'on a constaté qu'une température trop élevée limite la porosité des cristaux obtenus et donc, par la suite, ne favorise pas leur réaction avec l'acide fort. On peut s'assurer du degré de déshydratation par pesée. Le sel déshydraté est ensuite de préférence refroidi et maintenu en dessiccateur avant utilisation.

A noter que l'étape de déshydratation susmentionnée est optionnelle. On a en effet constaté qu'il suffit que le milieu contienne suffisamment d'agent déshydratant et/ou que l'on laisse le mélange « sécher » suffisamment longtemps pour éliminer toute l'eau. Il est toutefois préférable de partir d'un sel anhydre.

De manière tout particulièrement préférée, le sel métallique est un sel de métal bivalent anhydre. Dans ce cas, lors de la réaction avec l'acide fort, on va rapprocher 2 molécules de l'AHA et l'agent déshydratant (sel) formé va favoriser la formation du diester cyclique en captant les 2 molécules d'eau formées.

L'acide fort utilisé dans le procédé selon l'invention (et qui est de préférence également anhydre) doit répondre à deux conditions:
- son pKa doit être inférieur à celui de l'acide α-hydroxylé
- son sel avec le métal bivalent doit être hygroscopique c.à.d. être capable de réagir avec l'eau pour déplacer l'équilibre vers la formation du lactide; généralement, ceci se fait par la formation in situ d'hydrates. Afin de pouvoir en séparer le diester cyclique par distillation (sublimation: voir plus loin), l'hydrate formé in situ aura de préférence une température de décomposition supérieure a la température de distillation du diester cyclique.

Des acides qui conviennent bien sont l'acide ou l'anhydride (considéré comme un acide dans le cadre de l'invention) sulfurique ou phosphorique. Le 1^{er} possède comme avantage le fait que son pKa2 est également bas (1.9), de sorte qu'il est généralement également en dessous du pKa de l'acide α-hydroxylé (qui est par exemple de 3.86 pour l'acide lactique et de 3.83 pour l'acide glycolique) et qu'une seule mole d'acide par mole de sel métallique suffit. Il présente toutefois comme inconvénients, le fait de mener à un LD2 parfois coloré et de mener à des sous-produits difficilement valorisables (CaSO₄, MgSO₄). L'acide phosphorique (pka1 = 2.1) par contre ne mène pas à un problème de coloration et permet d'obtenir des sous-produits plus facilement valorisables (en particulier si à base de Ca ou Mg). Par contre, son pKa2 étant relativement élevé (7.2), il est généralement supérieur au pKa de l'acide α-hydroxylé. Dès lors, il est préférable de mélanger 2 moles de cet acide (ou une mole de l'anhydride correspondant soit P2O5) par mole de sel métallique bivalent.

Le mélange du sel métallique avec l'acide fort peut se faire de toute manière connue permettant d'atteindre un mélange intime des réactifs, par exemple dans un mortier, un mixer, un mélangeur-broyeur ou une extrudeuse. Cette dernière méthode donne de bons résultats en pratique car elle permet d'obtenir directement un produit (jonc ou granules si le jonc est découpé en sortie d'extrudeuse) ayant un grand rapport surface/volume permettant à la chaleur généralement utilisée pour favoriser la déshydratation, de pénétrer en son sein. Typiquement, l'extrusion d'un jonc de 2 à 5 mm de diamètre donne de bons résultats. Alternativement, le mélange issu d'un autre type de mélangeur peut être mis sous la forme d'un crêpe (film) ou de particules avant d'être soumis à maturation (réaction complète).

De préférence, on ajoute lentement l'acide au sel métallique sous peine d'avoir un échauffement trop violent qui pourrait mener à des problèmes de dégradation des produits de la réaction. Pour cette même raison, l'acide n'est de préférence pas ajouté en excès au milieu réactionnel.

Selon le choix des réactifs, un déshydratant (sel hygroscopique) additionnel (qui peut être ou non de même nature que le sel) peut être ajouté au milieu réactionnel soit pendant soit après le mélange des réactifs. En effet, lorsque le mélange est préparé en extrudeuse, sa consistance doit éventuellement être adaptée (pour obtenir une pâte effectivement extrudable). En outre, en fonction du pouvoir hygroscopique du sel (et notamment : du nombre de molécule d'eau que comprennent les hydrates qu'il est susceptible de former), un apport de sel externe peut être requis pour pouvoir réagir avec (absorber) toute l'eau de réaction. Dès lors, dans une variante particulièrement avantageuse, un sel issu d'une campagne de fabrication (batch) précédente peut être en partie réutilisé.

De même, il peut être avantageux d'ajouter au mélange (de préférence avant sa réaction), un autre sous-produit d'un batch précédent, par exemple de l'AHA qui n'aurait pas été transformé en diester. Une telle manière de procéder permet d'augmenter le rendement global de la réaction (l'acide produit à un batch étant recyclé au batch suivant).

Comme évoqué ci-dessus, le mélange (qui est généralement sous la forme d'une pâte) doit être soumis à maturation c.à.d. qu'il faut le laisser reposer le temps nécessaire pour que la succession de réactions chimiques menant au diester cyclique (libération de l'acide α-hydroxylé, suivi de sa dimérisation (en lactoyl-lactate ou L2A) et puis, de sa cyclisation (en LD2), et ce chaque fois avec libération d'eau) puissent être menées à terme. Il faut notamment bien veiller à évacuer l'eau de réaction du mélange sous peine de poly-condenser l'acide α-hydroxylé. Il faut également veiller à ne pas trop monter en température sous peine de distiller ledit acide et ce faisant, de l'extraire prématurément du milieu réactionnel. Des conditions identiques à celles du séchage des hydrates explicitées ci-dessus conviennent bien. De manière préférée, la pâte issue du mélange (sous forme de jonc, granules, film...) est déposée sur un lit de déshydratant pulvérulent durant cette étape de manière à ce que celui-ci puisse capter l'eau et s'assurer d'une part, que les réactions sont bien déplacées vers la droite et d'autre part, que l'AHA n'oligomérise pas. Cette poudre a également une fonction mécanique, à savoir d'empêcher la prise en masse lors de l'étape suivante de séparation du diester.

Dans le cas particulier où l'AHA est l'acide lactique, si l'eau n'est pas correctement éliminée du milieu réactionnel, celui-ci peut donc être le siège d'une certaine hydrolyse du LD2, soit en acide lactoyl-lactique (L2A), soit en monomère L1A. On peut limiter cet effet en ajoutant au mélange avant extrusion une quantité à déterminer de déshydratant, par exemple du produit secondaire d'un batch précédent que l'on aura desséché à 250°C par exemple. On peut aussi ajouter un déshydratant commercial, comme le sulfate de Mg anhydre.

A l'issue de l'étape de maturation, on est généralement en présence d'un solide dur mais friable qu'il est avantageux de broyer grossièrement (moyennant éventuellement un refroidissement préalable) pour faciliter la séparation du diester. En effet, l'eau de réaction ayant été progressivement (absorbée ou) captée par le sel hygroscopique, elle a provoqué l'agglomération des cristaux en cours de formation en une masse compacte. Ceci est en particulier le cas lorsque le déshydratant est l'hydrogéno-phosphate de Mg (c.à.d. lorsqu'on est parti de MgLa et d'une source d'ions phosphates), car celui-ci est un ciment magnésien bien connu. Le broyage de cette masse peut se faire de toute manière connue, de préférence jusqu'à obtenir des grains grossiers ayant une taille de l'ordre de 3 à 5 mm de diamètre. D'une part, ceci limite la résistance au transfert de matière dans le cas où le diester est séparé par distillation hors de la masse poreuse résiduelle. D'autre part, cette taille est caractéristique des granules d'engrais, de sorte qu'on pourra éventuellement utiliser ce sous-produit comme engrais sans devoir modifier les machines d'épandage.

Les grains ainsi obtenus sont alors généralement soumis à une séparation (extraction) du diester. Cette extraction peut avoir lieu par distillation (sublimation) sous vide ou courant de gaz inerte, pas extraction liquide-solide etc... Une distillation sous vide dans une colonne adéquate, de préférence rotative (pour créer un effet d'agitation) et chauffée (par exemple à une température finale (voir ci-dessous) de 120 à 240°C, de préférence de 150 à 200°C) donne de bons résultats). Dans le cas ou de l'AHA est ajouté au mélange, il peut être avantageux de travailler à plus haute température, par exemple à 180°C et plus, voire jusque quelques degrés en-dessous de la température de racémisation du diester, qu'il vaut mieux ne pas dépasser (et qui est de 250°C dans le cas du LD). Dans cette variante, il peut être avantageux de fluidiser les particules (favoriser leur mouvement relatif) par ajout de particules de déshydratant.

Pour favoriser l'élimination de l'eau résiduelle, il est préférable d'augmenter très lentement la température pendant la première partie de la distillation (jusqu'à 125°C).). Ainsi, on laissera par exemple la température augmenter a raison de 1 à 2°C/min, sans diminuer la pression sous 50 à 100 mbar. On sera ainsi à l'abri d'une évaporation prématurée de l'AHA (qui n'aurait donc pas eu l'occasion de se transformer en diester). Comme la volatilité de l'eau est largement supérieure à celle des produits organiques (diester, AHA (qui n'a pas entièrement réagi) et oligomères), le milieu réactionnel, acide, sera de plus en plus favorable au déplacement de l'équilibre en faveur du diester. Comme à ces températures, ces produits organiques sont généralement fondus, la mobilité nécessaire à la réaction est généralement acquise.

Pour cette étape de distillation également, il est avantageux de partir de granules durs et poreux, dont les pores sont bouchés initialement par le produit que l'on souhaite distiller (diester). Si les pores sont assez étroits, le liquide y sera maintenu par capillarité. Comme le chauffage se fait nécessairement de l'extérieur vers l'intérieur de chaque granule, sa surface a tendance à se dessécher au cours de la distillation. On pourrait dès lors utiliser pour cette distillation, des appareillages industriels (sublimateurs par exemple) chargés d'un mélange de granules partiellement desséchés et de granules frais, et où le risque de prise en masse serait inexistant. En même temps, les vapeurs issues de la distillation (ou sublimation) seraient condensées dans un échangeur de chaleur de forme appropriée.

A l'issue de cette étape, on aura donc recueilli d'une part, un sublimat dont on devra séparer les différents constituants (le diester principalement et comme impuretés, du monomère résiduel (AHA) et du dimère essentiellement), de préférence par fusion fractionnée ou distillation fractionnée (resublimation) ; et d'autre part, d'un résidu solide qui est principalement constitué du sel déshydratant et d'oligomères supérieurs de l'AHA.

L'opération de sublimation peut avoir lieu dans un échangeur de chaleur comprenant une enceinte et un faisceau de tubes parcouru par un fluide caloporteur qui est d'abord porté à une température légèrement (typiquement de 2 à 10°C) inférieure à la température de cristallisation de l'ester que l'on souhaite recueillir et où les vapeurs issues de la sublimation vont se condenser. On déposera ainsi sur chaque tube dudit faisceau refroidi, lors du chauffage des grains de solide susmentionné, une croûte annulaire d'ester brut, contenant en particulier des impuretés plus volatiles que l'ester lui-même, comme l'AHA correspondant.

Il suffit ensuite d'augmenter lentement la température du fluide caloporteur pour provoquer la fusion préférentielle d'un mélange riche en impuretés (puisque son point de fusion est plus bas que celui du diester pur). Ce mélange visqueux s'écoule lentement par gravité et est donc recueilli séparément dans la partie inférieure de l'échangeur. Il suffit ensuite d'augmenter suffisamment la température du fluide caloporteur pour fondre toute la couche cristalline restante, c.à.d. du diester purifié qui sera alors également recueilli dans la partie inferieure de l'échangeur et éventuellement soumis à un deuxième, voire à un 3^{ème} traitement similaire de cristallisation fractionnée, de préférence dans un autre appareil.

De manière particulièrement avantageuse, le traitement de sublimation a lieu dans un échangeur de chaleur comprenant une enceinte et deux faisceaux de tubes, de préférence verticaux et imbriqués ou en quinconce, et son enceinte est conçue pour pouvoir fonctionner sous vide. Ainsi, au lieu de réchauffer la couche d'ester brut déposée sur les tubes de l'un des faisceaux en commençant par sa zone la plus froide (en réchauffant le liquide de refroidissement), ce qui a pour effet d'affaiblir l'adhésion de ladite couche au faisceau de tubes, on peut envoyer dans le deuxième faisceau tubulaire un fluide caloporteur chaud qui chauffera, essentiellement par rayonnement, la partie encore chaude de la croûte d'ester brut. Le liquide visqueux résultant de la fusion de la dernière couche d'ester brut exposée ruissellera sur un support d'ester solide tandis que le vide sera maintenu de manière à permettre simultanément la distillation fractionnée des impuretés volatiles contenues dans le diester brut. Le diester purifié liquide sera recueilli dans la partie inférieure de l'échangeur, et les parties volatiles condensées par ailleurs.

Le même principe peut être appliqué pour bloquer l'arrivée dans la couche de diester brut d'impuretés non volatiles. A cette fin, on peut placer entre le sublimateur contenant les grains de solide à traiter et le ou les faisceaux de tubes, un petit échangeur de chaleur porté à une température supérieure à celle de la désublimation du diester mais inférieure à celle de condensation des oligomères de celui-ci, de sorte à recueillie un condensat d'oligomères à hydrolyser avant son recyclage.

Un appareillage de laboratoire unique qui permet d'effectuer toutes ces opérations dans la foulée est un appareillage de type four en verre de la marque BÜCHI. Un tel appareillage comprend un four rotatif de préférence transparent (par exemple en verre) ayant une forme généralement tubulaire comprenant des bulbes reliés par des partie de diamètre plus faible et qui est prolongé et/ou relié à un récipient de préférence également transparent et refroidi (condenseur). Un joint rotatif à l'extrémité froide du condenseur permet de relier l'appareil à une pompe à vide, l'eau libérée au cours de la réaction et non captée par le sel déshydratant étant condensée dans un piège à glace carbonique protégeant ladite pompe a vide.

Pour son utilisation dans le procédé objet de l'invention, cet appareil peut être utilisé comme suit: le sublimateur (ou bouilleur) est chargé de quelques dizaines de grammes de granules, et éventuellement d'une petite quantité de déshydratant pulvérulent. Les bulbes destinés à servir de condenseur sont assemblés au bouilleur. L'ensemble est glissé en place, le bouilleur seul se trouvant dans l'enceinte chauffante. Le condenseur est relié à la pompe à vide et l'ensemble est mis sous vide partiel. Bouilleur et condenseur sont mis en rotation, de sorte que les granules roulent les uns sur les autres, venant périodiquement en contact avec la paroi (chauffée) du bouilleur. On peut fixer la pression à 5 à 10 mbar et augmenter lentement la température jusque 125°C (1 à 2 °C/min). Pendant ce temps, de l'eau est évacuée et recueillie dans le piège à glace carbonique protégeant la pompe à vide.

On peut ensuite augmenter la température de consigne et provoquer la distillation des produits organiques occlus dans les granules. La quasi-totalité des vapeurs libérées se déposent dans le condenseur refroidi par l'air ambiant. Une petite partie s'échappe vers le piège à glace carbonique, tandis qu'une partie des composés moins volatils que le diester est condensée près du goulot de passage du bouilleur au condenseur.

On peut interrompre à volonté le fonctionnement de l'appareil, séparer le condenseur, le remplacer par un condenseur propre et ensuite redémarrer. On recueille ainsi toute la quantité d'ester brut sublimée depuis le dernier redémarrage, quantité que l'on peut peser et analyser.

La firme LIST commercialise des versions industrielles de sublimateurs-désublimateurs dont le principe de fonctionnement est semblable à celui décrit ci-dessus.

Alternativement, on peut utiliser un four vertical chargé de granules de déshydratant contenant le diester et fonctionnant de préférence à pression atmosphérique. Un gaz inerte chaud alimente l'appareil, de sorte qu'une onde de chaleur parcourt l'empilement. Ceci provoque la création d'une onde de transfert de matière telle que:
a.- le mélange organique est dans les granules avant le passage de l'onde
b.- il y a évaporation progressive lors du passage de l'onde
c.- les granules sont épuisés après le passage de l'onde.

La température initiale des granules est de préférence voisine de 100°C de manière à éviter toute condensation dans l'empilement. La température d'entrée du gaz inerte est de préférence inférieure à celle qui mènerait à la dégradation du diester, mais suffisante pour que la pression partielle de la vapeur du diester soit relativement élevée. Ainsi, dans le cas de la production de lactide, la température d'entrée du gaz inerte peut être comprise entre 160 et 210°C.

Le gaz inerte, chargé de vapeur, est dirigé vers un échangeur de chaleur à un seul faisceau tubulaire vertical où se déroule la désublimation. Comme ce désublimateur fonctionne à pression atmosphérique, on peut mener la purification du diester brut de manière légèrement différente de celle exposée ci-dessus.

En effet, une fois que tout l'empilement de granules est épuisé, et que tout le diester (impur) a été fixé sur les tubes de l'échangeur de chaleur sous forme de sublimat, on interrompt l'arrivée du gaz chaud à l'entrée de l'empilement. On admet alors un nouveau courant de gaz inerte à la base du désublimateur, gaz porté à température légèrement (de 5 à 15°C par exemple) supérieure à la température de fusion du diester. On provoque de la sorte la fusion de la couche extérieure (la plus chaude) du sublimat. Le liquide visqueux ainsi produit ruisselle à la surface du dépôt encore solide (et froid) et subit simultanément une désorption des composés plus volatils que le diester. Ces derniers sont condensés par ailleurs, le condensat étant recyclé vers une opération de préparation du sel de l'AHA. Le diester purifié est recueilli à la base de l'échangeur de chaleur. Enfin le gaz inerte débarrassé de vapeurs organiques est recyclé vers l'échangeur de chaleur où il est porté à la température voulue.

La présente invention est illustrée de manière non limitative par les exemples 1 à 5 ci-dessous.

### Exemple 1.

Dans cet exemple, on a en fait exécuté la réaction suivante :

MgLa2 + 2 H3PO4.H2O + Ca(H2PO4)2 → Mg(H2PO4)2.2H2O + LD + Ca(H2PO4)2.2H2O

Pour ce faire, on a procédé comme suit:

On a séché du lactate de magnésium dihydrate (acheté chez PURAC sous dénomination commerciale PURAMEX ® MG) par chauffage statique à pression atmosphérique, à 130°C, pendant 7 heures. Le sel déshydraté a ensuite été conservé dans un dessiccateur.

On a mélangé 20.2 g de ce lactate de Mg anhydre avec 17g de (Ca(H2PO4)2 (préalablement déshydraté à 110°C sous pression atmosphérique durant 12h à l'étuve). On a ensuite versé progressivement 23.2g d'acide phosphorique technique 85% sur la poudre, tout en mélangeant dans un mortier. A cette étape, l'acide phosphorique a libéré de l'acide lactique. La pâte obtenue était blanche et très épaisse.

Cette pâte a été mise à l'étuve durant 12h à 80°C sous pression atmosphérique. Le solide dur obtenu a été broyé en des grains que l'on a introduits dans un ballon à distiller. On a chauffé celui-ci dans un bain d'huile à 180°C et sous 4 mbar pendant 5h.

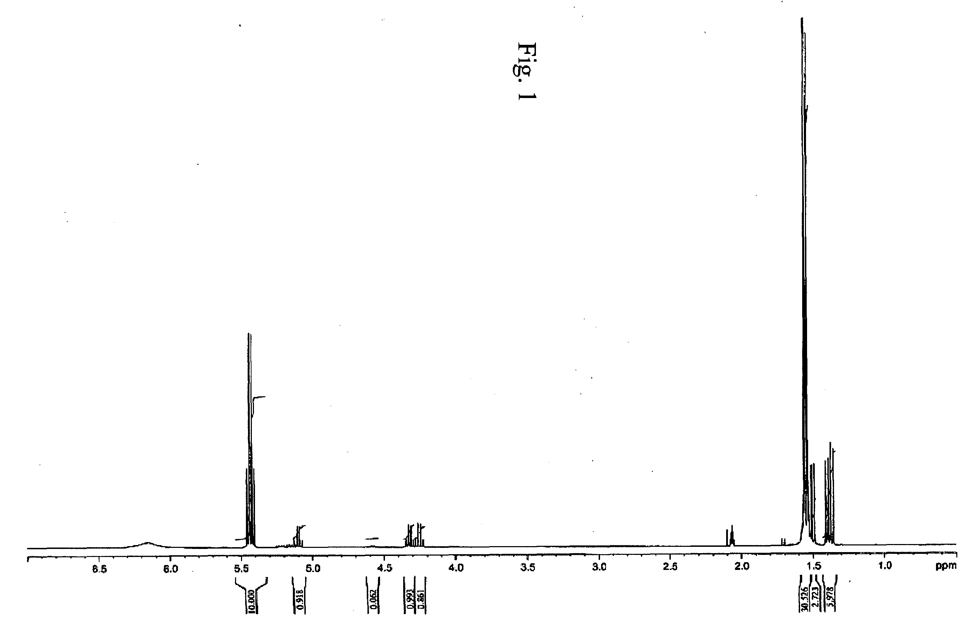

On a obtenu des cristaux sur la paroi de la verrerie. On a recueilli 2.7g de ces cristaux. Des analyses RMN (de Résonance Magnétique Nucléaire) ont été effectuées sur ces cristaux (voir figure 1) et ont montré que l'on avait extrait 2.7g de lactide pur à 85%, les 15% d'impureté étant 7.3% d'acide lactique et 8.4% de dimère ouvert. On obtient donc un rendement en masse de l'ordre de 20%

Voici le protocole utilisé pour les analyses RMN :
- on a prélevé 20 mg de l'échantillon
- on l'a mis en solution dans l'acétone
- on a dispersé les insolubles de la solution à l'aide d'ultrasons
- on a filtré la solution pour en ôter les insolubles avant de l'analyser par la technique RMN.

### Exemple 2.

Cet exemple a pour but de démontrer que la distillation permet effectivement de séparer l'acide du diester.
Pour ce faire, on a procédé comme suit:

13.9g de pentahydrate de CaLa et 10.5g d'acide lactique (LA, 88%) ont été mélangés dans un Pétri. On a déshydraté partiellement le mélange sous pression atmosphérique à 110°C durant 90 min. Le solide obtenu, qui pesait 19.3g, a été broyé finement et on a recueilli 18.8 g de poudre à laquelle on a mélangé 12.2g d'acide phosphorique (solution aqueuse à 85%) dans un mortier.

La pâte résultante a été extrudée à l'aide d'une seringue pour former une sorte de jonc (spaghetti) pesant 26.9g que l'on a séché sur Pétri durant 3.5 h sous pression atmosphérique à 110°C. Le solide séché et broyé grossièrement en particules de 2 à 5 mm pesait alors 23.4g.

On y a ajouté 2g d'un batch précédent (réalisé dans des conditions identiques) et chargé un appareil de distillation de type BÜCHI (tel que décrit précédemment) avec les 25.4g de solide ainsi obtenu.

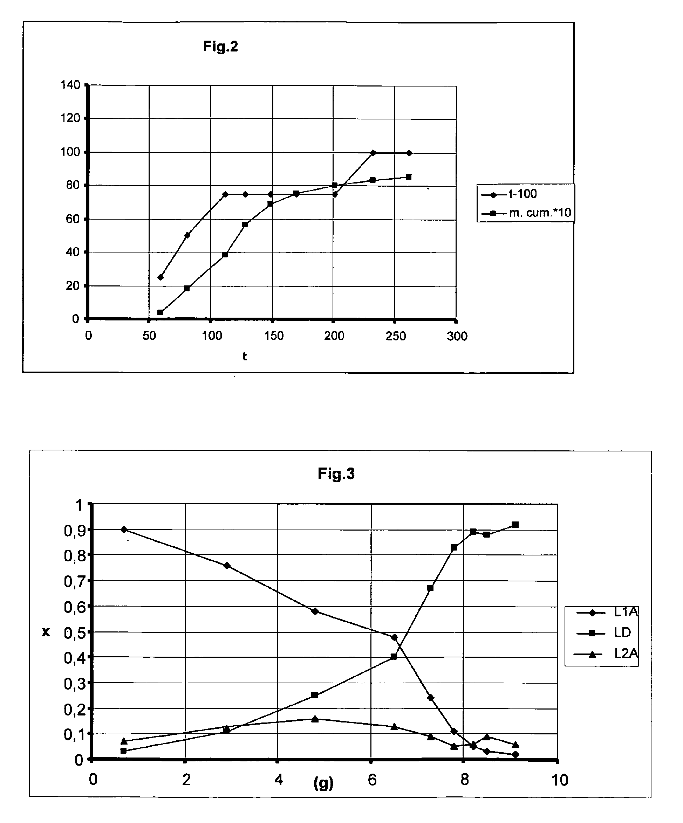

L'appareil a été mis sous vide (5 à 10 mbar) et la température de consigne a évolué comme indiqué à la figure 2 (qui représente en fait en ordonnée, cette température - 100 °C).

A intervalles réguliers, on a arrêté la rotation de l'appareil et le chauffage pour pouvoir recueillir du sublimat (à proprement parler, au début de l'opération c'est un condensat), que l'on a pesé et mis à l'abri de l'air avant l'analyse. La figure 2 donne également en ordonnée, l'évolution du produit par 10 de la masse de sublimat cumulée (m. cum., en g) au cours du temps.

On a analysé la composition des fractions consécutives de sublimat par RMN (dissolution dans l'acétone).

Le résultat de ces analyses figure dans le tableau en annexe et dans la figure 3 (où l'on a porté en abscisse, le poids cumulé de sublimat et en ordonnée, la fraction pondérale de chaque constituant).

On peut y voir que les 9g recueillis au total contiennent 8.08g de groupes lactate alors qu'on on en avait introduits 10.18g au départ. Le taux de recouvrement du lactate est donc de 79%.

En mélangeant toutes les fractions on obtiendrait un lactide brut titrant 39% en poids de lactide.

Cette figure montre clairement qu'au début de la séparation, c'est essentiellement de l'acide lactique que l'on recueille au condenseur, alors que l'acide lactoyl-lactique et le lactide n'apparaissent que plus tard. Il est intéressant de noter que les trois derniers échantillons recueillis titrent environ 90% en lactide, ce qui suggère que la purification ultérieure, décrite plus haut, sera aisée.

### Exemple 3.

Cet exemple a pour but de démontrer que les réactifs de départ ne doivent pas forcément être anhydres.
Pour ce faire, on a procédé comme suit :

28.1 g de pentahydrate de lactate de calcium ont été dissous dans 100 g d'eau dans un récipient de 250 ml, sous agitation à 60°C, puis 12.8 g d'acide phosphorique 85% a été ajouté. Après 80 minutes d'évaporation il restait une masse en partie cristallisée qui a été transférée dans un mortier et mélangée à 12.5 g d'acide phosphorique à 85%. On a pu récupérer 49.4 g de pâte dont la consistance a permis l'extrusion à l'aide d'une seringue dont le diamètre de l'embout était de 5.2mm.

46.9 g du jonc ainsi produit ont été portés à l'étuve à 50°C pendant 18 heures, ce qui a réduit le poids à 36.7 g. Après broyage grossier, 33.7 g des granules obtenus ont été chargés dans le bouilleur du minisublimateur BUCHI de type B-585 Kugelrohr.

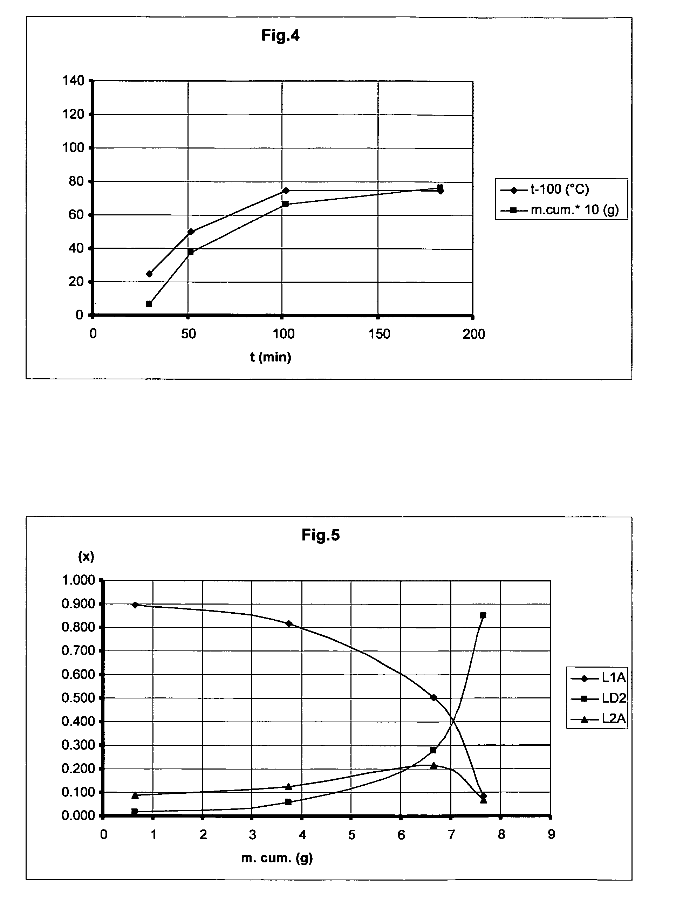

L'appareil a été mis sous vide (5 à 10 mbar) et la température de consigne a progressivement augmenté comme indiqué à la figure 4 (qui représente en fait en ordonnée, cette température - 100 °C).

A intervalles réguliers, on a arrêté la rotation de l'appareil et le chauffage pour pouvoir recueillir du sublimat (à proprement parler, au début de l'opération c'est un condensat), que l'on a pesé et mis à l'abri de l'air avant l'analyse. La figure 4 donne également en ordonnée, l'évolution du produit par 10 de la masse de sublimat cumulée (m. cum., en g) au cours du temps.

L'analyse par RMN a permis de mesurer les concentrations des divers constituants, soit en considérant les protons compris dans le groupe CH3, soit en considérant les protons liés à un carbone portant un groupe alcool ou un groupe ester. On rapporte à la figure 5, les moyennes de ces concentrations (x ou fraction massique) pour chaque échantillon.

On peut y voir qu'au début de la distillation, on libère surtout de l'acide lactique (L1A), puis de l'acide Lactoyllactique (L2A) et du lactide (ou dilactide, LD2), et ce n'est qu'à la fin de la séparation que le lactide devient dominant dans le sublimat. De ce fait, si on mélangeait toutes les fractions de sublimat, on obtiendrait un mélange dont les titres massiques seraient:
L1A : 0.631
LD2 : 0.225
L2A : 0.144

### Exemple 4

Cet exemple a pour but de démontrer que l'on peut ajouter au mélange, un sous-produit de réaction d'un batch précédent (ici, de l'acide lactique).
Pour ce faire, on a procédé comme suit:

On a chargé un récipient de 250 ml de 55 g de pentahydrate de lactate de calcium, 42.1 g d'acide lactique 88% et 25 g d'eau. Après ébullition sous agitation pendant une heure à pression atmosphérique, la bouillie obtenue a été transférée dans un mortier et 50.2 g d'acide phosphorique 85% y ont été ajoutés. Après mixage, le mélange pâteux a été extrudé en un spaghetti que l'on a laissé vieillir à température ambiante pendant quelques jours, jusqu'à devenir un solide que l'on a grossièrement broyé en granules de 2 à 5 mm de diamètre moyen.

Pour éviter que ces granules ne s'agglomèrent lors de la séparation dans le mini-sublimateur, on a ajouté à la charge une petite quantité de dessicant (5 g de poudre de monocalcium de phosphate anhydre, obtenu par broyage du résidu d'un batch précédent) pour une charge de 25 g de granules.

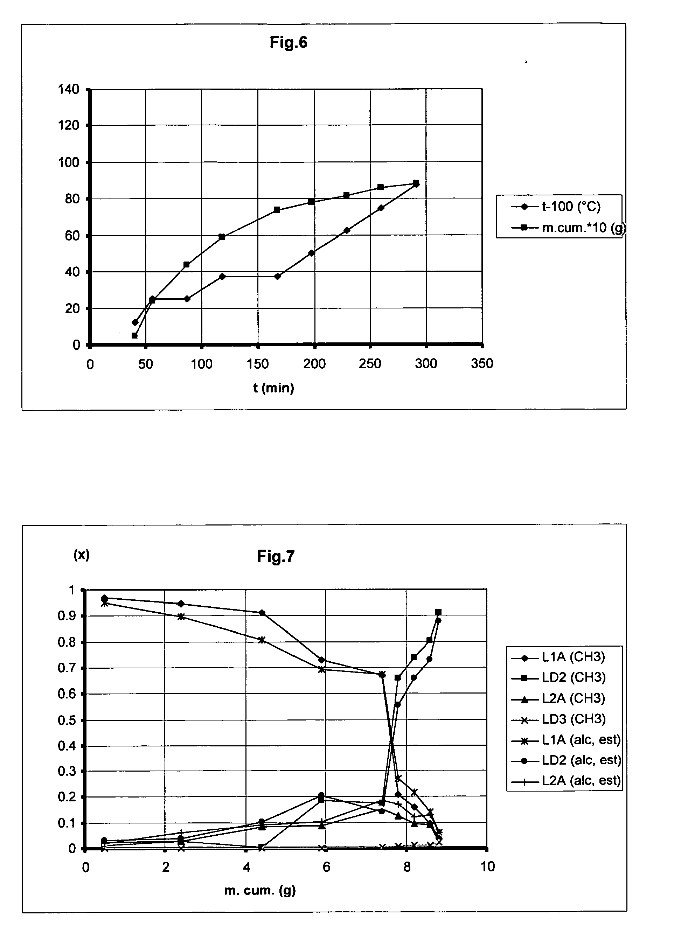

La figure 6 montre que, sous une pression de 5 à 10 mbar, la séparation peut être menée à température relativement basse. Mais dans ce cas (figure 7), la concentration en lactide du sublimat ne devient importante qu'en fin de séparation.

A la figure 7, on a porté les concentrations obtenues par analyse des signaux de RMN par les deux voies décrites dans l'exemple précédent.
Les titres massiques du mélange de toutes les fractions de sublimat seraient:
L1A : 0.747
LD2 : 0.170
L2A : 0.082
LD3 : 0.001

### Exemple 5

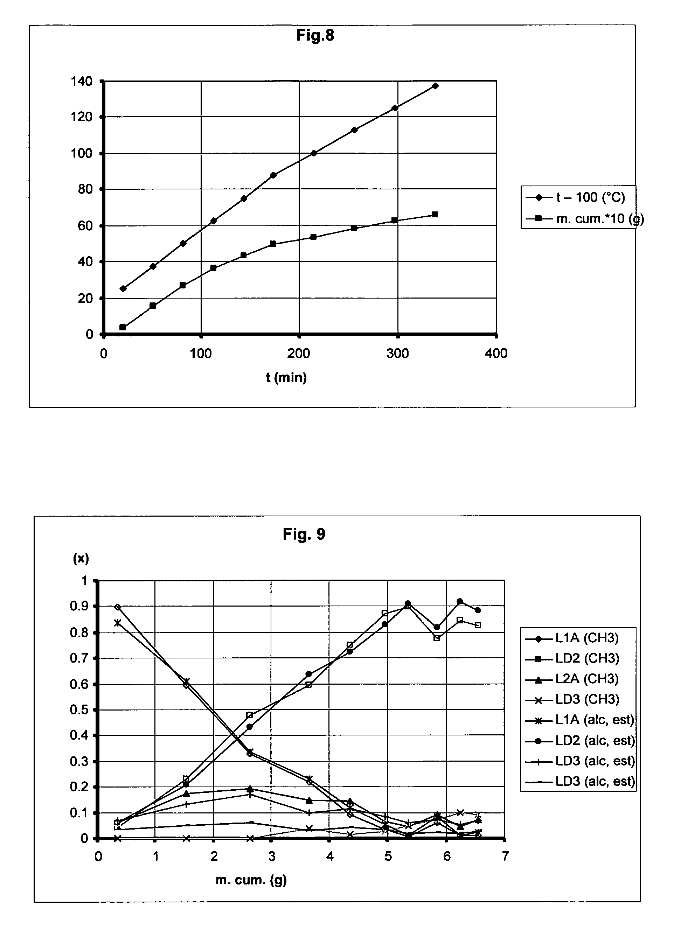

L'exemple 4 a été répété à température plus élevée (comparer les figures 8 et 6). Dans ce cas, la teneur en lactide du sublimat a augmenté beaucoup plus rapidement que dans l'exemple précédent (comparer les figures 9 et 7), et on a constaté l'apparition de nouveaux pics de RMN qui ont été attribués au trilactide (LD3, le triester cyclique).
Les titres massiques moyens du sublimat seraient ici:
L1A : 0.300
LD2 : 0.552
L2A : 0.115
LD3 : 0.033
Il semblerait donc que de manière surprenante, le fait de sublimer à haute température (typiquement, au-delà de 180°C) permette de favoriser l'obtention d'un titre moyen en diester élevé.

| Fraction N° | | Poids | Poids cumulé | L1A | LD | L2A | L1A | LD | L2A | Lactate | | |
|---|---|---|---|---|---|---|---|---|---|---|---|---|
| | | g | g | x | x | x | g | g | g | ex-L1A | ex-LD | ex-L2A |
| | 1 | 0,7 | 0,7 | 0,9 | 0,03 | 0,07 | 0,63 | 0,021 | 0,049 | 0,504004 | 0,021 | 0,043556 |
| | 2 | 2,2 | 2,9 | 0,76 | 0,11 | 0,13 | 1,672 | 0,242 | 0,286 | 1,33761 | 0,242 | 0,254223 |
| | 3 | 1,9 | 4,8 | 0,58 | 0,25 | 0,16 | 1,102 | 0,475 | 0,304 | 0,881606 | 0,475 | 0,270223 |
| | 4 | 1,7 | 6,5 | 0,48 | 0,4 | 0,13 | 0,816 | 0,68 | 0,221 | 0,652805 | 0,68 | 0,196445 |
| | 5 | 0,8 | 7,3 | 0,24 | 0,67 | 0,09 | 0,192 | 0,536 | 0,072 | 0,153601 | 0,536 | 0,064 |
| | 6 | 0,5 | 7,8 | 0,11 | 0,83 | 0,05 | 0,055 | 0,415 | 0,025 | 0,044 | 0,415 | 0,022222 |
| | 7 | 0,4 | 8,2 | 0,05 | 0,89 | 0,06 | 0,02 | 0,356 | 0,024 | 0,016 | 0,356 | 0,021333 |
| | 8 | 0,3 | 8,5 | 0,03 | 0,88 | 0,09 | 0,009 | 0,264 | 0,027 | 0,0072 | 0,264 | 0,024 |
| | 9 | 0,6 | 9,1 | 0,02 | 0,92 | 0,06 | 0,012 | 0,552 | 0,036 | 0,0096 | 0,552 | 0,032 |
| Somme | | | | | | | 4,508 | 3,541 | 1,044 | 3,606426 | 3,541 | 0,928004 |

| | | | | | | | | | | | | |
|---|---|---|---|---|---|---|---|---|---|---|---|---|
| x = fraction pondérale L1A = acide lactique LD = dilactide cyclique L2A = lactoyl-lactate | | | | | | | | | | | | |

## Revendications

1. - Procédé pour la fabrication d'un diester cyclique d'un acide α-hydroxylé comprenant les étapes suivantes :
- on mélange un sel d'un métal et d'un acide α-hydroxylé, avec un acide fort dont le pKa est inférieur à celui de l'acide α-hydroxylé et dont le sel avec le métal est hygroscopique
- on laisse réagir le mélange à une température et pendant une durée suffisantes pour obtenir le diester cyclique dispersé dans le sel hygroscopique.

2. - Procédé selon la revendication précédente, où acide α-hydroxylé est l'acide lactique.

3. - Procédé selon l'une quelconque des revendications précédentes, où le métal est le Ca ou le Mg.

4. - Procédé selon la revendication précédente où le sel substantiellement anhydre est obtenu par déshydratation au moins partielle de dihydrate de MgLa2 (lactate de Mg) ou de pentahydrate de CaLa2 (lactate de Ca).

5. - Procédé selon l'une quelconque des revendications précédentes, dans lequel le sel est un sel d'un métal bivalent anhydre.

6. - Procédé selon l'une quelconque des revendications précédentes, dans lequel l'acide fort est l'acide phosphorique.

7. - Procédé selon la revendication précédente, dans lequel on mélange 2 moles d'acide phosphorique par mole de sel métallique bivalent.

8. - Procédé selon l'une quelconque des revendications précédentes, dans lequel le mélange est soumis à l'action de sel hygroscopique additionnel pendant et/ou après sa fabrication.

9. - Procédé selon l'une quelconque des revendications précédentes, dans lequel de l'acide α-hydroxylé est ajouté au mélange.

10. - Procédé selon l'une quelconque des revendications précédentes, dans lequel le mélange est extrudé.

11. - Procédé selon l'une quelconque des revendications précédentes, dans lequel le mélange est soumis à maturation jusqu'à l'obtention d'un solide.

12. - Procédé selon la revendication précédente, dans lequel le solide est broyé et puis soumis à distillation/sublimation.

13. - Procédé selon la revendication précédente, dans lequel la sublimation a lieu dans une enceinte comprenant un faisceau de tubes parcouru par un fluide caloporteur, et selon lequel ce fluide est d'abord porté à une température inférieure à la température de cristallisation du diester cyclique pour déposer sur ce faisceau une croûte annulaire de diester contenant des impuretés plus volatiles; ensuite, la température du fluide est augmentée graduellement pour provoquer la fusion d'un mélange riche en impuretés que l'on recueille dans la partie inférieure de l'enceinte ; et enfin, la température du fluide est augmentée suffisamment pour fondre le diester purifié que l'on recueille également dans la partie inférieure de l'enceinté.

14. - Procédé selon la revendication 12, dans lequel la sublimation a lieu dans une enceinte qui comprend deux faisceaux de tubes et qui est conçue pour pouvoir fonctionner sous vide, et selon lequel le fluide caloporteur du premier faisceau est d'abord porté à une température inférieure à la température de cristallisation du diester cyclique pour déposer sur ce faisceau une croûte annulaire de diester contenant des impuretés plus volatiles ; ensuite, on porte le fluide caloporteur du deuxième faisceau à une température suffisante pour provoquer la fusion de cette croûte en surface et on met l'enceinte sous vide pour provoquer la distillation fractionnée des impuretés volatiles qu'elle contient ; enfin, on recueille le diester purifié dans la partie inférieure de l'enceinte.

15. - Procédé selon la revendication 13 ou 14, selon lequel on place entre le solide et le ou les faisceaux de tubes, un échangeur de chaleur porté à une température supérieure à celle de la désublimation du diester mais inférieure à celle de désublimation des oligomères de celui-ci, de sorte à y condenser et recueillir les oligomères en vue de leur recyclage.
